Europäisches Patentamt

European Patent Office

Office européen des brevets

(19)

(11) Publication number: **0 138 625**
**A2**

(12) **EUROPEAN PATENT APPLICATION**

(21) Application number: **84307147.3**

(22) Date of filing: **17.10.84**

(51) Int. Cl.⁴: **H 05 G 1/26,** H 05 G 1/60,
G 06 F 15/68, G 01 T 1/29,
A 61 B 6/00

(30) Priority: **17.10.83 US 542384**

(43) Date of publication of application: **24.04.85**
**Bulletin 85/17**

(84) Designated Contracting States: **DE FR GB NL**

(71) Applicant: **PICKER INTERNATIONAL, INC., 595 Miner
Road, Highland Heights Ohio 44143 (US)**

(72) Inventor: **Mattson, Rodney A., 6532 Melshore Drive,
Mentor Ohio 44092 (US)**
Inventor: **DeMeester, Gordon D., 1613 Dennis Drive,
Wickliffe Ohio 44092 (US)**

(74) Representative: **Pope, Michael Bertram Wingate, Central
Patent Department Wembley Office The General Electric
Company, p.l.c. Hirst Research Centre East Lane,
Wembley Middlesex HA9 7PP (GB)**

(54) Radiographic system.

(57) A radiographic system comprising a source of penetrative radiation (10) and a detector (14) having a plurality of energy sensitive detector elements for producting electrical signals in repsonse to the radiation. Means are provided for actuating the source (10) so as to direct radiation through a subject (P) onto the detector (14). Means are also provided for moving the detector (14) relative to the subject (P) whilst exposing a number of detector elements to the radiation passing through the subject (P). Time delay and integrate circuitry is associated with the detector elements so as to operate on the signals produced by the elements so as to improve their signal to noise ratio. Imaging circuitry is provided for producing a representation of the internal structure of the subject corresponding to the pattern of radiation incident on the detector elements.

Radiographic System

This invention relates generally to the field of diagnostic imaging, and more particularly to digital scan projection radiography, and systems and methods for improving noise characteristics and manageability of data production rates in such systems.

Radiographic systems and techniques have long been used in medical diagnostics. In a typical conventional radiographic system, a patient is positioned between an x-ray source and a radiographic screen which serves as a radiation detector. X-rays from the source pass through the patient's body and emerge in a pattern to fall incident upon the screen, which produces an image describing the pattern. The image provides radiologists with information relating to the internal structure or condition of the patient's body.

In conventional radiography, the screen comprises a layer of radiographic film sensitive to electromagnetic radiation in both visible and invisible portions of the spectrum. The layer of film is sandwiched between x-ray intensifier screens. The image is produced by processing the film after x-ray exposure.

Conventional radiographic systems, while simple and economical, and having good spatial and temporal resolution characteristics, have several disadvantages. Conventional radiographic systems have quite limited power to detect low contrast levels in an image. The display is rather inflexible. Storage and retrieval of images is expensive and cumbersome.

Also, conventional radiography can produce only a shadow image, in which portions of the body of an inter-

est can sometimes obscure the body portions desired to be imaged.

More recently, digital radiographic systems have been proposed which produce image information whose quality and flexibility are in some ways superior to that produced in conventional radiographic imaging. Digital radiography systems are often categorized by the geometry of x-ray beams they employ. Systems considered most promising include so-called "area beam" systems and "scan projection radiography" (SPR) systems.

In a typical area beam digital radiographic system, a divergent cone of x-rays is directed through the patient's body toward the input face of an image intensifier tube. The image intensifier tube responds to the pattern of x-rays incident on its input face to produce at an output face a smaller, bright visible light image corresponding to the pattern.

In such systems, known as computed fluoroscopy, a high quality video camera views the intensifier tube output face and produces video signals corresponding to the light image. These video signals are then digitized. In their digital form, the signals are susceptible to many types of useful digital processing, such as contrast enhancement. Additionally, the digital signals are susceptible of storage and retrieval in very convenient form, such as on tape or disc, and can be transmitted readily to remote locations.

Moreover, digital systems lend themselves well to techniques of temporal and energy subtraction, which if employed can reduce or eliminate the disadvantage of conventional shadow imaging by actually removing unwanted portions of the body from the image.

According to another proposal, area beam digital radiography can be practiced by the use of a solid state detector comprising an arrangement of discrete detector elements. Each detector element responds to incident energy to produce an analog electrical signal representing that incident energy which caused its production. The respective electrical signals collectively represent the pattern of x-rays emergent from the patient's body. See "Digital Fluorography: A Technology Update, 1981, published by The General Electric Company, page 6.

Circuitry coupled to each of the detector elements amplifies and digitizes these analog electrical signals. Digital processing circuitry and equipment responds to the digitized signals to produce a digital representation of a diagnostic image.

While digital radiography represents a significant advance in some respects, problems do exist. For instance, because the entire screen is simultaneously actuated to detect x-rays, some radiation scatter, which always occurs, is detected by the entire screen and in effect "blurs" the image. Scatter radiation falls in a rather diffuse fashion onto the detector. This causes the production of spurious signals not indicative of the image pattern of the primary radiation energy emergent from the patient's body.

Also, veiling glare can degrade the image.

It is clear that as one reduces the size of each discrete detector element, image resolution improves, since each detector element represents a smaller pixel, or portion, of the image. Since, however, separate electrical circuitry must be associated with each individual detector element, the cost and complexity of a detector rises sharply with the number of individual detector elements used. A limit on maximum resolution is reached when the detector elements become so small that electronics are no longer available with sufficient compactness to be coupled to each element.

4

Another problem with this type of digital radiography stems from the fact that the entire large field is illuminated simultaneously. This results in generation of data simultaneously for the whole detected image. This high rate of data generation requires more expensive and sophisticated digital processing equipment than would be the case if data were produced more slowly.

Data transfer rates are limited by disc transfer rates and by availability of data compression hardware. Though efforts have been made to improve data rates, this problem still exists.

Scan projection radiography eliminates or reduces some of the problems inherent in area beam radiography. Scan projection radiography (SPR) utilizes a smaller detector array than area beam radiography, and acquires data for large area imaging by synchronous scanning movement of the source and detector array relative to the patient.

In one proposed, type of SPR, the x-ray beam from the source is collimated by a suitable slit defining structure into a thin spread beam lying substantially in a plane. The detector assembly comprises a generally elongated array of discrete detector elements. Each detector element can suitably comprise, for example, a photodiode with a phosphor layer optically coupled to a receiving light sensitive surface facing the radiation source.

Mechanism is provided for effecting relative movement between the source-detector arrangement and the patient. Such movement can take the form of pivoting the radiation source about an axis extending through the focal point of x-ray beam generation, combined with a corresponding synchronous movement of the detector, either along an arc of a circle or a line, such that all the detector elements remain, during this motion, aligned with the collimated spread beam which is directed through the patient.

In the course of this movement, the spread beam passes through a succession of planes of the patient's body. The detector simultaneously produces information relating to the pattern of spread beam radiation which emerges from the body and is seen by the detector as a transversely moving line. The spread beam illuminates simultaneously all the detector elements of the elongated detector array.

The detector output is repeatedly read out to generate a succession of signal sets. Each set represents radiation emergent from the patient's body along the line defined by the location of the spread beam at the instant of detector readout. Together, these individual "line scans" make up an entire image.

Scan projection radiography provides some advantage over area beam digital radiography, but at the expense of some disadvantages.

It is well known, for instance, that line scan projection radiographic systems can provide improved detection of low contrast subjects by reducing the effect of scatter radiation. This advantage results from the fact that both the spread beam of radiation and the detector array used in SPR are relatively thin and the detector does not detect scatter radiation from regions of the patient's body outside the line being instantaneously examined. Thus, there is less opportunity than in area beam radiography for scatter to produce spurious signals.

Scatter grids can additionally reduce scatter, to some extent, but at the expense of output signal strength.

Scan projection radiography does, however, have some significant disadvantages. The flux of radiation from the source which is available for detection by the

6

detector array is limited by the thinness of the collimated spread beam and, of course, by the output from the x-ray source. With less radiation falling upon the detector elements, these elements can become x-ray quantum limited, with a consequent degradation of contrast in the resulting image.

Present technology, as discussed above, requires a channel of preamplifier electronics for each detector element. In order to increase resolution, larger numbers of smaller detector elements are required. The cost of providing this increased number of electronic components becomes uneconomical. Achievement of resolution above a certain maximum is not attainable because it can require a not yet developed level of electronic compactness.

According to another proposal, a digital radiography system is provided utilizing a stationary X-ray source and detector. A collimated fan beam from the source is directed toward an image intensifier. The image intensifier output is transmitted to a detector having a rectangular matrix of individual detector elements. In scanning, the patient is moved along a linear path between the stationary source and image intensifier. Time delay and integrate circuitry, comprising charge coupled device (CCD) circuitry, is provided to enhance signal to noise ratio at the detector.

This proposal, however, suffers from some disadvantages. In the time delay and integrate function, it is very important that in shifting charge from one element to another, almost no charge be lost, i.e., the charge transfer must be extremely efficient. At the same time, in order to provide sufficient dynamic range, each element of the system must be required to handle a relatively large amount of charge.

The charge coupled device (CCD) circuitry of this proposed scanner loses charge shifting efficiency when relatively large amounts of charge are shifted. As a result, data becomes attenuated in the multiple shifting operations of the time delay and integrate function. This is directly opposed to the advantage of the time delay and integrate circuitry which is to enhance the signal to noise ratio of the information.

The method of scanning employed, wherein the patient moves along a linear path between the source and the detector, causes a resolution loss because, in such motion, adjacent ray paths are not colinear.

It is an object of this invention to provide a high resolution, efficient digital radiographic system and method which maintains the reduced scatter of scanned projection radiography, but which utilizes many times more efficiently the radiation available from the source, for producing image data with improved signal to noise characteristics, and which system also produces data at a sufficiently slow rate to be manageable with digital processing equipment of reasonable cost.

Disclosure of the Invention

The disadvantages of the prior art are reduced or overcome by a radiographic system comprising a source of penetrative radiation and a detector having a plurality of energy sensitive detector elements for producing electrical signals in response to that radiation. The system also includes means for actuating the source to direct radiation through a subject to emerge therefrom in a pattern incident on the detector. Means is provided for moving the detector relative to the subject while simultaneously exposing a plurality of the detector elements to the radiation emergent from the subject. Time delay and integration circuitry is associated with the detector elements in order to enhance the signal to noise ratio of the image representing data from the detector. Imaging circuitry responds to these enhanced signals and produces a representation of internal subject structure corresponding to the pattern of emergent radiation.

In accordance with one aspect of this invention, the time delay and integrate circuitry comprises bucket brigade register circuitry. The bucket brigade circuitry has noise characteristics superior to those of the charge coupled device circuitry, notwithstanding that the latter is often considered more advanced and "state of the art" than is the older bucket brigade type. Additionally, the bucket brigade circuitry has greater capability than does the charge coupled device circuitry for efficiently shifting relatively large quantities of charge from one element to the next and thus minimizing the loss of charge. This feature is extremely important in this environment, where high rates of charge shifting can, if done inefficiently, significantly degrade image quality.

In accordance with another aspect of this invention, the penetrative radiation source is pivoted about an axis to scan the radiation across the patient's body. Simultaneously, the detector assembly is also moved in order to maintain alignment with the pivoting energy beam. This technique yields higher resolution than the prior art technique of passing the patient along a linear path between a stationary source-detector pair.

In accordance with the further aspect, the use of an image intensifier is avoided, the radiation emergent from the patient being directly incident on the detector. Thus, any image degradation attendant upon the use of the intensifier is eliminated.

In accordance with another aspect, the detector consists of a number of light sensitive elements such as photodiodes. Material is interposed near the receiving surfaces of the detector elements for converting X-rays to light energy which can be sensed by the detector. Fiber optics light pipe structure may be utilized in coupling the light converting means to the detector elements, minimizing signal loss and other optical problems.

Direct coupling of the light conversion means to the detector elements is, however, preferred. The effects of missing detectors at the abutments between detector arrays should be minimized.

The disadvantages of the prior art are reduced or eliminated, and the object of this invention attained, by a medical diagnostic imaging system embodying this invention and including an x-ray source and an x-ray detector assembly having at least one elongated array of individual detector elements. Each detector element responds to the occurrence of x-ray energy in a unique region to produce and maintain an electrical signal which represents x-ray energy incident on the corresponding detector region.

The system also includes scanner apparatus for effecting arcuate motion of the detector array at a predetermined velocity, with respect to a subject to be examined. Circuitry and apparatus actuates the x-ray source to direct x-ray energy through the subject, causing a plurality of the detector elements to respond to the x-rays simultaneously during the course of the longitudinal movement of the detector array.

Circuitry coupled to the detector elements iteratively shifts and integrates the electrical signals maintained by the individual detector elements. This electric signal shifting is in a longitudinal direction along successive detector elements, at velocity equal and opposite to the predetermined velocity of longitudinal detector motion.

Output circuitry transmits a succession of the shifted and integrated electrical signals from one of the detector elements, upon each signal's arrival at that element.

Imaging circuitry responds to the transmitted, shifted and integrated electrical signals to produce a tangible representation of internal structure of the subject.

The system embodying the invention as described above maintains the good resolution and low susceptibility to effects of scatter which is characteristic of scan projection digital radiography systems, due in part to the elongate configuration and relatively small size of the detector array. The array images a relatively wide expanse of the subject by means of physical motion during x-ray exposure, rather than by simultaneously imaging the entire field, which increases the effect of scatter.

The inventive system, while incorporating this advantage of SPR, at the same time overcomes a prior disadvantage of such systems, i.e., the relatively low level of x-ray flux available to individual detector elements. This improvement is made possible by the simultaneous exposure of many of the detector elements during detector motion, coupled with the shifting and integrating circuitry. The simultaneous exposure of all of the detector element row gathers more data for equal scan rates, than would exposure of only a single element of the row. The shifting and integrating circuitry, by iteratively moving and accumulating the electrical signals in a velocity equal and opposite to that of detector motion, eliminates spatial ambiguity in the significance of signals, which would otherwise result from the multiple element simultaneous exposure during detector movement. The shifted and integrated signal present at any moment at any given detector element represents x-ray passing through only a single location in the subject's body.

The combination of multiple, relatively prolonged, simultaneous detector element exposure and of operation of the shifting and integrating circuitry (compensating for ambiguities which would otherwise be caused by detector motion) results in a very significant improvement in the signal to noise ratio of image data produced by the detector array and transmitted by the output circuitry.

The system of this invention also overcomes the previous disadvantage of area beam radiographic systems, i.e., the production of image data at excessively high rates. The present system produces digital data at a slower, more manageable rate. The output circuitry need not be designed to accept data essentially simultaneously from each of the individual detector elements. Instead, the output circuitry need only accept data as it arrives, after having been shifted and integrated many times, at a single one of the detector elements.

According to a more specific aspect of the invention, the x-ray source comprises an x-ray tube whose structure defines a focal spot for a beam of x-rays generated by the tube when actuated. The apparatus for simultaneously exposing a plurality of individual detector elements includes mechanism for pivoting the x-ray tube about its focal spot and for moving the detector array synchronously with the pivoting of the tube to maintain at least a portion of the detector array continuously illuminated by x-ray.

Another specific feature includes structure defining a fore slit interposed between the x-ray tube and the subject for collimating the x-ray energy emanating from the tube into a relatively thick spread beam. Another specific feature involves the use of structure defining an aft slit which is positioned between the subject and the detector array for further collimating the x-ray spread beam and for intercepting scatter. The system further includes mechanical apparatus for moving the slit defining structures synchronously with the pivoting motion of the x-ray tube and the motion of the detector array. This synchrony of motion maintains the slit defining structures and detector array mutually aligned, and positioned in the beam emanating from the tube.

In previous SPR equipment, the spread beam typically was collimated to a degree of thinness such that it would illuminate only a single column of individual detector elements, relative to scanning motion direction. Since the thin beam did not simultaneously illuminate pluralities of detector elements in the direction of detector motion, such systems could not take advantage of shifting and accumulating circuitry for enhancing the signal to noise ratio of image data from the detector array.

A further specific feature of an embodiment of this invention comprises an encoder for sensing instantaneously the location of the movable detector array. The encoder is coupled with servo equipment to synchronize the pivoting motion of the x-ray tube with detector array motion.

In a preferred embodiment, the detector array includes a vertical column of adjacently arranged imaging chips. Each imaging chip includes a rectangular matrix of individual detector elements which together constitute a large number of parallel rows of individual detector elements, similar to the single row described in connection with the more general embodiment. Each row of detector elements is provided with shifting and integrating circuitry which is analogous in design and operation to that described above in connection with the more general embodiment which comprises a detector array having a single horizontal row of detector elements.

By utilizing a collection of these image chips, each including many parallel rows of detector elements, a detector array is provided which can scan a portion of the patient's body several inches in width.

In accordance with a more specific embodiment, each individual detector element comprises a photodiode which is responsive to visible light incident on a receiving surface thereof to produce an analog electrical signal representing a characteristic of the light incident on the detector element.

In accordance with another specific feature, the shifting and integrating circuitry comprises a different storage capacitor associated with each of the detector elements, and circuitry connecting the storage capacitors of adjacent detector elements into parallel chains extending along each row of detectors. The shifting and integrating circuitry also comprises clocking circuitry for timing the shifting and integration of the electric charge packets stored on the capacitors.

Further in accordance with this specific feature the circuitry comprises bucket brigade circuitry. Bucket brigade circuitry is particularly useful in this environment, in that such circuitry is more capable of efficiently shifting electric charge than is more expensive charge coupled device (CCD) circuitry which is often today considered more current as state of the art.

CCD circuitry can nonetheless, under certain conditions, be used as an embodiment of the charge shifting circuitry.

Where CCD circuitry is so used, it is sometimes preferable to employ detector elements having a rectangular shape, but whose dimension in the direction of charge shifting along the row is substantially less than its orthogonal dimension. Such a configuration employing rectangular detector elements with unequal side dimensions enables the use of many more detector elements per unit length along the direction of charge shift.

The use of a larger number of detector elements in the direction of charge shifting helps compensate for the more limited charge handling capacity of the CCD circuitry.

In a more specific aspect of the embodiment, each of the image chips can suitably comprise a 64 x 64 matrix of individual detector elements, where bucket brigade circuitry is used.

Where CCD circuitry is used, each image chip can advantageously comprise a 64 x 256 array of individual detector elements. In this embodiment, each detector element has a rectangular shape, the narrower dimension, in the direction of charge shifting, being approximately 1/4 its orthogonal dimension. In this way, a 64 x 256 matrix can comprise an image chip having an overall square array of detector elements.

Several other specific aspects of the embodiment of this invention reside in the apparatus and manner in which the detector elements are rendered responsive to the occurrence of x-rays. In one such embodiment, the detector elements are simply covered by a unitary phosphor layer which converts incident x-ray to visible light. Where a need exists to better protect the detector elements, such as photodiodes, from effects of direct radiation, a mosaic of thicker crystals may be used in place of the phosphor screen.

Where it is considered advantageous to physically remove the image chips from direct x-radiation, a coherent fiber optic coupling device can be used. Solid state devices with thin depletion layers are relatively insensitive to ionizing radiation. Devices with thin depletion layers designed for charge transfer applications can be directly coupled to the light converters. The input of the fiber optic coupler can include a phosphor layer for converting the x-ray to light. The light subsequently is conducted by the fiber optic coupling to the image chip, which can be remotely located, or oriented such that radiation does not fall incident on its operative receiving surfaces.

These and other aspects of the present invention will be understood in more detail by reference to the following description, and to the drawings, in which:

Brief Description of the Drawings

Figure 1 is a pictorial drawing illustrating the major components of a digital scan projection radiography system in which the present invention is suitably employed;

Figure 2 is a generalized block diagram of the system of Figure 1;

Figure 3 is an elevational view of a portion of the system of Figure 1;

Figures 4A-4D are partly pictorial, partly block illustrations exemplifying in simplified form an operation sequence of the system of Figure 1 incorporating the present invention;

Figure 5 is a detailed elevational view illustrating a portion of the system of Figure 1;

Figures 6 and 7 are side views illustrating embodiments of the portion of the system shown in Figure 5;

Figure 8 is a pictorial view of an alternate embodiment of a portion of the system of Figure 1;

16

Figures 9 and 10 are pictorial views illustrating geometrical component relationships and modes of operation of a portion of the system of Figure 1;

Figures 11 and 12 are detailed top views illustrating a portion of the system shown in Figure 1;

Figure 13 is a detailed block diagram illustrating a portion of circuitry of the system of Figure 1; and,

Figure 14 is a graphical timing diagram illustrating operation of a portion of the system of Figure 1.

Best Mode for Carrying out the Invention

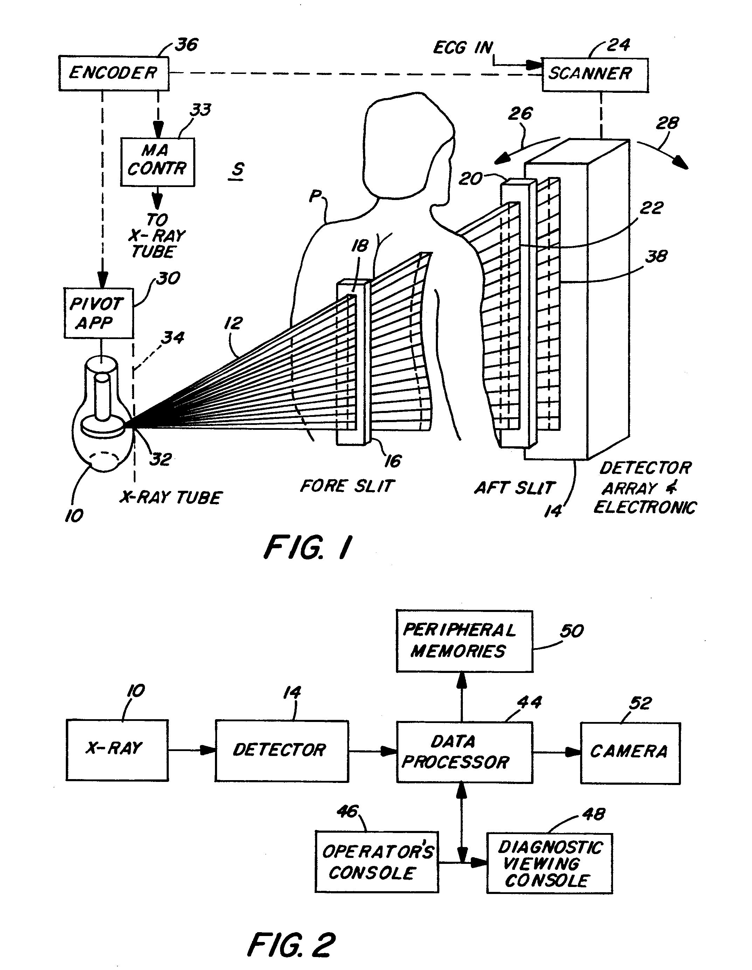

A system S for performing digital scan projection radiography (SPR) is illustrated in general form in Figure 1. The system S directs a pattern of x-rays through a patient P and produces, from information borne by the x-ray pattern emergent from the patient's body, a tangible representation, generally in the form of a visible image, describing the internal structure or condition of the patient's body.

The system S incorporates an x-ray source 10 for directing a beam of x-ray energy illustrated as a collection of rays 12 through the patient P and onto a detector assembly 14. A first collimator structure 16 defines a generally vertical fore slit 18 for collimating the x-rays emanating from the source into a spread beam lying generally within a vertical plane. A second collimator structure 20 defines an aft slit 22 located between the patient and the detector assembly for further enhancing this collimation.

Gantry structure (not shown) connects the collimators 18, 20 to the detector assembly 14 to maintain mutually constant alignment between the collimators and the detector.

Mechanical scanner apparatus 24 is coupled to the detector assembly 14 to move the detector along an arcuate path defined by arrows 26, 28. The gantry structure maintains alignment between the collimator structures 16, 20 and the detector during detector motion.

Pivoting apparatus 30 is coupled to the x-ray source. The apparatus 30 pivots the source synchronously with detector arcuate motion to continuously track the detector 14 and the mutually aligned collimators 16, 20.

The x-ray source 10 comprises an x-ray tube, and associated power circuitry (not shown) for electrically actuating the tube to produce the x-rays (in pulsed or continuous mode) emanating from a focal spot 32 defined by the structure of the tube. The pivoting motion effected by the pivot apparatus 30 causes the tube to pivot about a vertical axis 34 extending through the focal point 32.

It is believed preferable to couple the detector to the master drive of the scanner apparatus and to control the tube to follow, since detector positioning is more critical than tube position.

An encoder 36 is coupled to the scanner apparatus 24 and produces a signal indicating the instantaneous position of the detector 14 along its arcuate path described by the arrows 26, 28. The output of the encoder 36 is directed to the pivot apparatus 30 for synchronizing the pivoting motion of the x-ray tube 10 with the arcuate motion of the detector 14 and collimators 16, 20, to maintain continuous alignment between the x-ray beam, collimators and detector assembly during scanning motion.

The scanner apparatus can be appropriately gated by a physiological signal, such as ECG, or with a signal indicating timing of administration of a contrast agent (see Fig. 1). Temporal subtraction studies can also be done with sufficiently rapid retrace between scans, which, as explained below, is possible with this system.

An example of a type of encoder apparatus is described in U.S. Patent No. 4,015,129, issued on March 29, 1977 to Manring, et al, incorporated by reference.

The encoder 36 may also be coupled to the m.a. (current) control 33 of the x-ray tube 10. The encoder can adjust the tube m.a., and hence, the intensity of x-ray output, as a function of the location of the detector along its scanning path. In the embodiment described here, the tube m.a. can be controlled to decrease as a function of the degree of detector displacement from the middle position along its scanning path. Thus, where the patient's body is less thick, i.e. near its right and left sides, x-ray output is reduced to maintain a more uniform x-ray flux at the detector throughout its scan.

The detector 14 includes an array of individual detector elements, generally arranged in the region of an elongated slot 38 of the detector assembly 14. The structure and arrangement of the detector elements is described in detail below. Each of the detector elements responds to light energy (generated by x-rays as described below) incident on a receiving face thereof to produce an electrical signal which represents a characteristic of the x-ray which caused the production of the electrical signal.

In operation, the detector, collimators and x-ray tube are moved to the left as in the direction illustrated by the arrow 26 to prepare for a scan. In performing a scan, the x-ray tube 10 is actuated to produce x-ray energy. The scanner apparatus 24 and pivot apparatus 30 is operated to synchronously scan the vertical spread beam of x-rays from left-to-right in Figure 1 across the patient's body. During this scanning motion, the detector elements of the detector assembly 14 produce analog electrical signals. The electrical signals produced by the array of detector elements is rapidly and repeatedly sampled to produce sequential representations of image data corresponding to closely spaced vertical lines through the patient's body over the course of the scan.

The electrical signals are then digitized and processed to produce the desired patient imaging.

Figure 2 illustrates a generalized block diagram of the system of Figure 1. In Figure 2, the x-ray source 10 directs x-rays to the detector assembly 14. Signals from the detector 14 are transmitted to a data processor 44 which digitizes and processes the electrical signals. In response to commands from an operator's console 46, the data processor 44 produces various types of tangible representations of internal body structure of the examined patient. In one mode, the data processor actuates a diagnostic viewing console 48 to produce directly a visible image of the patient's internal body structure which can be immediately employed by a radiologist for medical diagnostic purposes.

In another mode, the data processor 44 stores digital information representing patient image data in one or more peripheral memories 50. Optionally, a camera 52 can be coupled to the data processor.

Digital scan projection radiography systems are described in the following publications and their bibliography, all of which are specifically incorporated by reference: Mattson, R. A. et al "Design and Physical Characteristics of a Digital Chest Unit", S.P.I.E. Vol. 314, Digital Radiography (1981); Digital Radiography, the General Electric Company, 1980; Digital Fluorography, The General Electric Company, 1981.

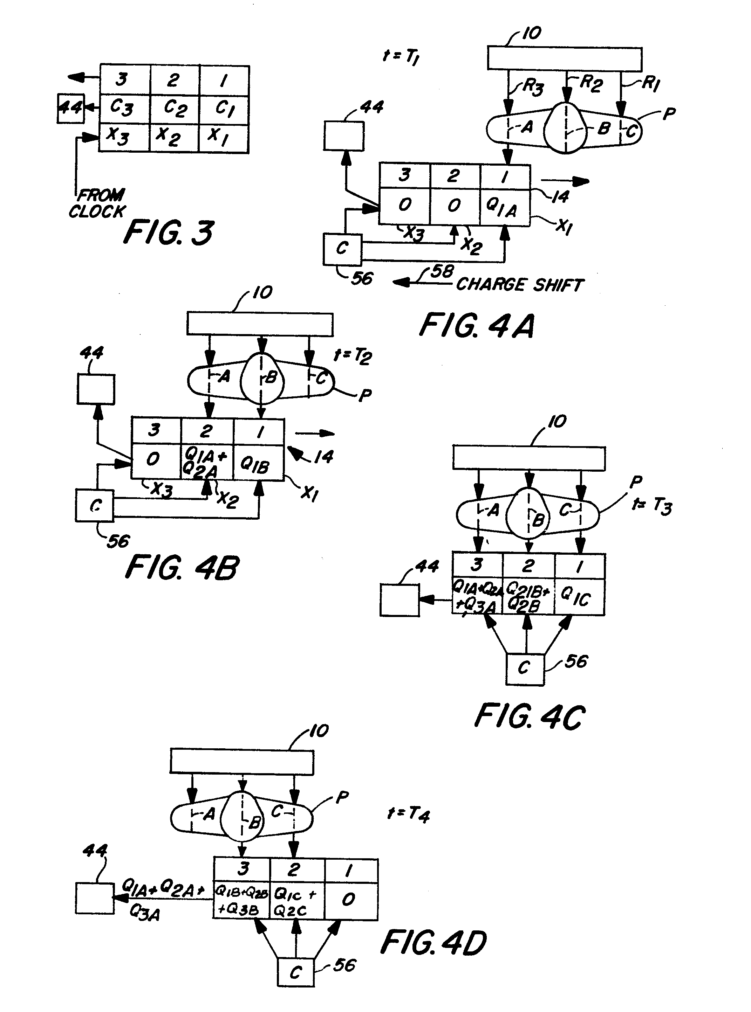

Operation of the detector assembly 14 embodying the present invention is believed initially best described by means of a highly simplified example. This example is illustrated mainly by reference to Figures 3 and 4A, 4B 4C and 4D.

The operation of the detector assembly 14 is in accordance with a technique which is sometimes referred to as "time delay and integrate" (TDI). Figure 3 illustrates a greatly simplified form of detector element arrangement incorporated into the detector assembly 14. Figure 3 shows a row of three individual detector elements 3, 2, 1. The detector elements 3, 2, 1 are arranged in an adjacent linear configuration extending horizontally, with respect to Figure 1, across a portion of the slot 38 of the detector assembly 14. In practice, there are far more than three detector elements in each such row, and also there are a great number of horizontal rows of such detectors distributed vertically along the entire height of the slot 38. For purposes of explanation, however, only this single row of detectors will be initially considered.

Each of the detector elements 3,2,1 is adapted to generate a signal which is a function of the intensity of the radiation falling thereon. Each detector element is coupled to a different element $X_3$, $X_2$, $X_1$, of a parallel in, serial out shift register SR by way of associated storage capacitors $C_3$, $C_2$, $C_1$. The shift register R thus receives parallel input data from the detector elements 3,2,1. Each of the signals from the detector elements is stored in a respective storage element of the register SR.

The shift register SR is controlled by a clock signal from clock circuitry 56. In response to each clock pulse, data in the register elements $X_3$, $X_2$, $X_1$, is shifted one element to the left with respect to Figure 3.

When the appropriate clocking signal is present, the signal stored in the storage element $X_1$, for example,

is shifted to storage element $X_2$. The signal stored in $X_2$ is shifted to $X_3$. The signal stored in $X_3$ is shifted out of the register, to be processed in a manner described in more detail below.

As a given signal is shifted through the register, it passes through the storage elements corresponding to each of the detector elements 3,2,1. While the signal is present at each of these storage elements, it is augmented by any signal then received from the associated detector element.

Consider, for example, a charge signal $Q_1$ corresponding to detector element 1, and held in $X_1$. In response to the clock signal, the signal $Q_1$ will be shifted to element $X_2$. While there, it will be augmented by a signal $Q_2$ generated in response to radiation incident on detector element 2. Thus the signal present in register element $X_2$ will be $Q_1 + Q_2$.

In response to the next clock pulse, $Q_1 + Q_2$ will be shifted to register element $X_3$. While there, it will be augmented by a signal $Q_3$ produced by detector element 3, to become $Q_1 + Q_2 + Q_3$.

Referring now to Figure 4A, there is shown a simplified representation of an x-ray source 10. The x-ray source 10 emits x-radiation through the patient P toward the detector assembly 14. In this simplified diagram, the x-ray source 10 may be thought of as a source of parallel rays of x-ray emitted simultaneously in a downward direction as shown in Figure 4A. The lateral dimension of the source 10 is greatly exaggerated, relative to the patient, for purposes of simplicity and clarity. In actuality, however, the lateral dimension of the x-rays directed to the patient is defined by the thickness of the spread beam which penetrates the patient after collimation by the collimator 16.

Each of the detector elements 3, 2, 1, as described above, is coupled to respective elements $X_3$, $X_2$, $X_1$ of the register SR. The charge signals are produced by the detector elements, which may suitably comprise photodiodes. The magnitude of each charge signal represents the intensity of the x-ray falling on the detector element which caused the production of the charge.

The clocking circuitry 56, in response to signals from the encoder 36, provides clocking signals to the shift register such that in response thereto the charge signals are caused to iteratively shift to the left along the shift register elements as shown by the arrow 58 in Figure 4A. Additionally, each time a charge is shifted one element to the left, it becomes summed, or integrated while there, with any charge then produced by the detector element corresponding to the register element to which the charge has just been shifted.

The design and construction of the elements 3, 2, 1 and of the associated clocking, capacitor and register circuitry, will be described in detail below.

Figures 4A-4D describe operation of the source-detector at four equally spaced points in time, i.e., $T_1$, $T_2$, $T_3$ and $T_4$. In the course of this operation, the source 10 and detector 14 are synchronously moved to the right as shown in Figure 4A at a predetermined velocity. This motion may be continuous, or may be in stepwise fashion.

The charge shifting actuated by the clocking circuitry has velocity equal and opposite to that of the detector motion.

In this example, we shall consider imaging at three points in the patient's body, i.e., along lines designated A, B, C in Figure 4A.

At time t = $T_1$, the detector is located such that x-ray energy penetrates the patient along the line A and falls incident upon the detector element 1. This occurrence results in the production of a charge signal $Q_{1A}$ at the register element $X_1$.

The signal designated as $Q_{1A}$ is that signal developed by individual detector element 1 as a result of x-rays passing through line A of the patient P. Signals developed by other detector elements by virtue of x-rays passing through other lines through the patient's body will be referred to by analogous notation.

Subsequent to the acquisition of charge $Q_{1A}$ at register element $X_1$, the detector moves, as shown in Figure 4B, to a location, at time $t = T_2$, at which x-rays passing through patient line B are incident upon detector element 1, and x-rays through patient line A are now incident on detector element 2.

During the time of motion between the positions illustrated in Figures 4A and 4B, respectively, the clocking circuitry 56 has operated to shift the signal $Q_{1A}$ from element $X_1$ to element $X_2$. Now, upon arriving at the position shown in Figure 4B, element $X_1$, (which now contains no signal, its signal $Q_{1A}$ having been shifted to element $X_2$) acquires a new charge $Q_{1B}$, and element $X_2$ also acquires an added charge signal referred to as $Q_{2A}$. However, since charge packet $Q_{1A}$ was already shifted to element $X_2$ by the clocking circuitry, the total charge signal at element $X_2$ is now $Q_{1A} + Q_{2A}$.

The process continues. The detector moves farther to the right such that at time $t = T_3$, the location is as shown in Figure 4C. Additionally, during the move between $T_2$ and $T_3$, the clocking circuitry 56 has again shifted the signals by one element to the left.

It is now seen that x-rays from the source 10 are incident on each of detector elements 3, 2, 1 along lines A, B and C, respectively.

Since the previously held charge $Q_{1B}$ has been shifted from element $X_1$ to element $X_2$, element $X_1$, just before time $t = T_3$, contains no charge. At $t = T_3$, however, element $X_1$ acquires a charge $Q_{1C}$. Element $X_2$ acquires an additional charge $Q_{2B}$ which is summed with charge $Q_{1B}$. Element $X_3$, to which charge signals $Q_{1A} + Q_{2A}$ have already been shifted, now acquires an additional new charge $Q_{3A}$.

Between time $t = T_3$ and time $t = T_4$, the clocking circuitry 56 again causes the iterative and accumulative shifting of charge by one element to the left. This time, however, the charge reaching element $X_3$, the last in the row, is transmitted to further processing circuitry 44. The quantity of charge shifted, $Q_{1A} + Q_{2A} + Q_{3A}$, represents three accumulated charge packets, each of which is the result of a response of a different one of the elements 3, 2, 1 to x-ray energy passing through the same line A of the patient's body. Since exposure to each of the three elements 3, 2, 1 caused the production of the charge shifted to the processor 44, that charge is approximately three times greater in magnitude than it would have been, had only one exposure to a single element been used to provide data as to the radiation penetrating the patient along the line A, assuming equal detector scanning velocity. Thus, the signal to noise ratio of the charge signal is considerably improved.

It will be noted that at time $t = T_4$, the charge now present on element $X_3$ is $Q_{1B} + Q_{2B} + Q_{3B}$, and thus represents a value of charge produced in three exposures of three different elements in response to radiation passing through the same line B of the patient's body. Subsequently, the charge present at element $X_3$ at time $t = T_4$ will also be transmitted to the data processor 44 for integration into the image representing data.

After the completion of the full transit of the source detector pair from left to right as shown in Figures 4A-4D, the motion stops and the X-ray source is deactuated. All elements are initialized. When another such scan is to be initiated, the source detector pair is moved to the left and stopped at the appropriate initial location. The x-ray source is reactuated, and source-detector motion is again begun.

It can be seen from this example that, if the number of elements in the horizontal row were multiplied many times, the effect on the signal to noise ratio of the charge signals actually transferred to the data processing equipment would be improved still further.

Note that the processing circuitry 44 need not accept data simultaneously from all the elements. Rather, data is transferred only from element $X_3$, and only as rapidly as the clocking frequency of the shifting circuitry. This relatively slow data transfer rate enables the use of relatively simple and economical processing circuitry.

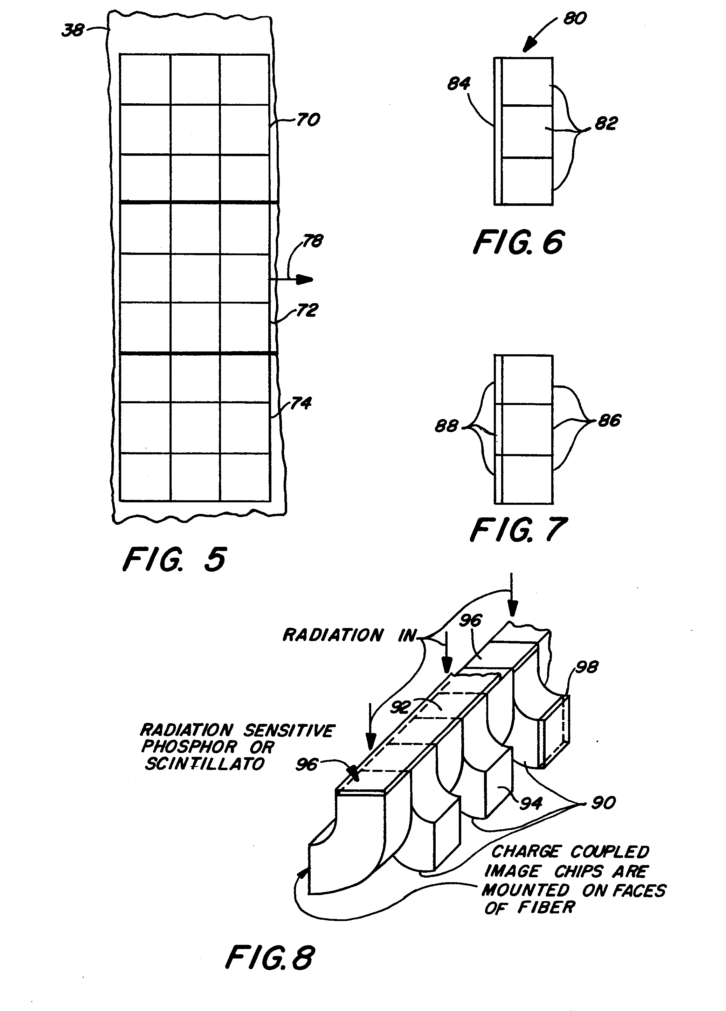

In the preferred embodiment, the detector assembly comprises a plurality of relatively large imaging chips, each of which includes a rectangular matrix of individual detector elements. Such an assembly of image chips, designated 70, 72, 74, is illustrated in simplified form in Figure 5.

The image chips are disposed in a vertical column, with respect to the system of Figure 1 and, as such, are moved during scanning in a direction illustrated by the arrow 78 in Figure 5.

Each of the horizontal rows of individual detector elements in each image chip is coupled to circuitry for causing their cooperative operation in a sequence analogous to that described in connection with Figures 4A-4D.

In Figure 5, each image chip is illustrated, for sake of simplicity, as comprising a 3 x 3 array of individual detector elements. In practice, and as described in more detail below, each image chip has an overall square configuration having a dimension of 8 millimeters on a side, and comprises either a 64 x 64 or 64 x 256 array of individual detector elements.

As mentioned above, in the preferred embodiment each individual detector element comprises a photodiode. The photodiode is responsive to visible light falling upon a receiving surface to produce an electrical charge signal representing a characteristic of the visible light which caused the production of the signal.

Figures 6-8 illustrate various structures for interfacing the visible light sensitive photodiodes with incident x-ray energy emergent from the patient's body.

Figure 6 shows a side view of 3 x 3 image chip 80 including photodiodes 82. Overlying the photodiodes 82 is a phosphor layer 84 made of one of the known materials for converting incident x-ray to visible light. When x-rays impinge from the left as shown in Figure 6, the phosphor layer 84 luminesces and the resulting visible light pattern is detected by the individual detector element photodiodes 82.

Sometimes it is desirable to provide protection from excessive exposure of the silicon photodiodes and associated circuitry to direct x-radiation. A structure suitable for such purpose is illustrated in Figure 7.

Figure 7 is another side view of an image chip illustrating three photodiodes 86. In this embodiment, the phosphor layer of Figure 6 is replaced by a mosaic of individual scintillation crystalline material illustrated at reference character 88. The crystalline material comprises one of many suitable materials known to those of ordinary skill as having the property of converting x-radiation to visible light, along with appropriate mechanical and chemical properties. The crystals 88 can be somewhat thicker than the phosphor layer 84 shown in Figure 6. The thicker crystals are more absorptive of radiation than is the phosphor layer, and thereby provide some shielding effect for the photodiodes, while still facilitating their operation.

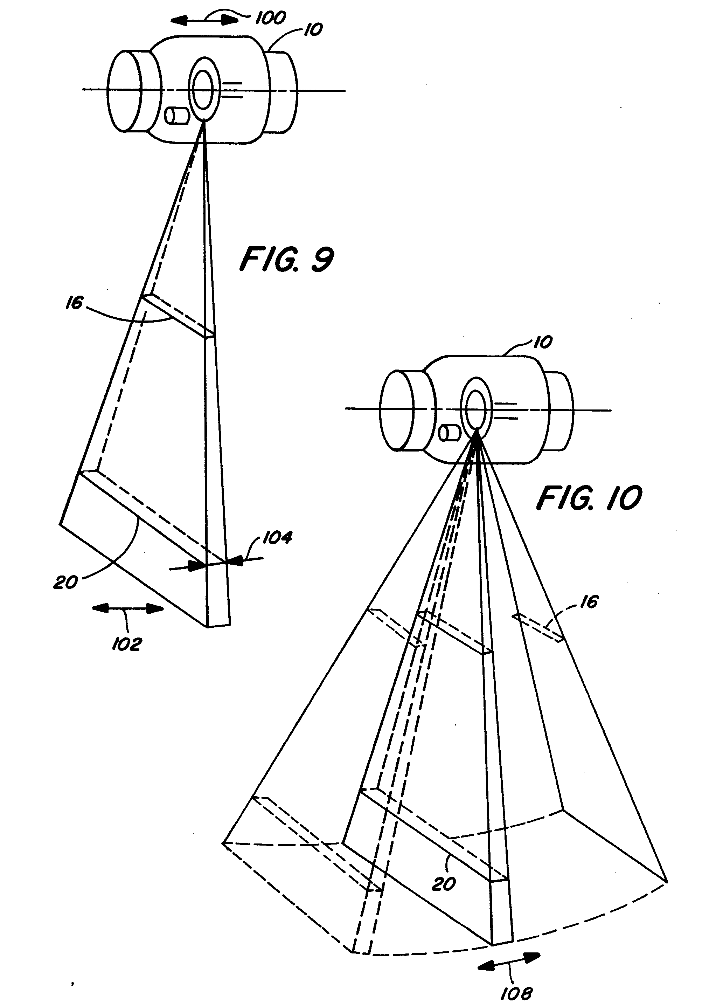

Another arrangement, providing still greater radiation protection for the image chips, is shown in Figure 8. The principal feature shown in Figure 8 is a series of coherent fiber optic coupling elements illustrated for example at reference character 90. Each optical coupling element has an input face such as 92 and an output face such as shown at reference character 94. The input face and output face 92, 94 are configured to be geometrically congruent with the square face of each of the imaging chips. Suitable fiber optic materials include quartz, glass and plastic.

A phosphor layer, such as 96, overlies each of the input faces of the coupling elements 90. The output face of each coupling element is optically coupled to the receiving face of an image chip such as shown at reference character 98.

By the use of coherent fiber optic coupling, the image chips can be disposed at a location which is not in the path of x-radiation from the source 10, or at a location at which its receiving face is oriented in a direction such that the incident radiation from the source does not fall upon that surface.

The embodiment of Figure 6 is preferable where cost is paramount, and is suitable for uses such as in airport baggage inspectors.

Figures 9 and 10 illustrate certain hardware implementations for achieving the moving spread beam. In Figure 9, for example, both the x-ray source 10 and the detector assembly are translated along parallel linear paths indicated by arrows 100, 102 in Figure 9. Preferably, the spread beam is 8 millimeters in thickness at the input surface of the detector elements. This dimension is indicated by arrows 104 in Figure 9.

Another hardware implementation is illustrated in Figure 10. In this embodiment, the detector moves generally along an arcuate path indicated by arrows 108 in Figure 10. The source 10 can be pivoted about its x-ray focal spot to cause the x-ray beam to track the detector. Alternately, where the cone of x-rays generated by the x-ray tube is sufficiently divergent, the x-ray tube may be retained in a stationary disposition, the actual x-ray energy striking the detector being collimated by moving the collimating slit defining structures 16, 20, illustrated in Figures 9 and 10, along an arcuate path.

A requirement peculiar to the system embodying this invention is that data is acquired in one x-ray spread beam scanning direction only. This direction is from left to right, as shown in Figure 1. The mechanical scanning apparatus should be capable of rapid retrace between production of sequential images. It is mechanically practicable to produce retrace at velocities much higher than scanning velocities.

Tests have shown that suitable scanning velocities at the detector face are in the neighborhood of up to about 10 inches per second.

Additionally, it is possible to read out data by scanning in both directions. In such an embodiment, this is accomplished by reversing scanning motion, simultaneously reversing shift register clocking direction, and by reading data out of the opposite end of the register.

In embodiments employing a moving spread beam of x-rays, it has been found preferable that the detector itself should be coupled to the master scanning drive mechanism. The source, and the slit defining collimators should be the follower elements, since their instantaneous position is less critical than that of the detector. An encoder, such as shown in Figure 1, coupled to the detector, provides timing pulses to control the position of the source and/or collimating slit defining structures. The pulses output from the encoder are also coupled to the clocking circuitry 56 to control the timing of charge stepping of the charge shifting and accumulating circuitry. Such an arrangement will assure good synchronization between detector motion in one direction and charge motion in the opposite direction at the same average velocity. Very accurate synchronization of charge and detector motion is possible since the moving charges on the detector do not possess inertia and the charge shifting can be implemented with a favorable frequency response.

Retrace and scanning rates, and hence frame repetition rate, is substantially limited by only the mechanical considerations, not by optics or electronics. Tests have shown that frame repetition rates of at least about 30 per minute are feasible, using a frame size of 20 in. X 20 in. Faster rates are obtainable with smaller scan arcs.

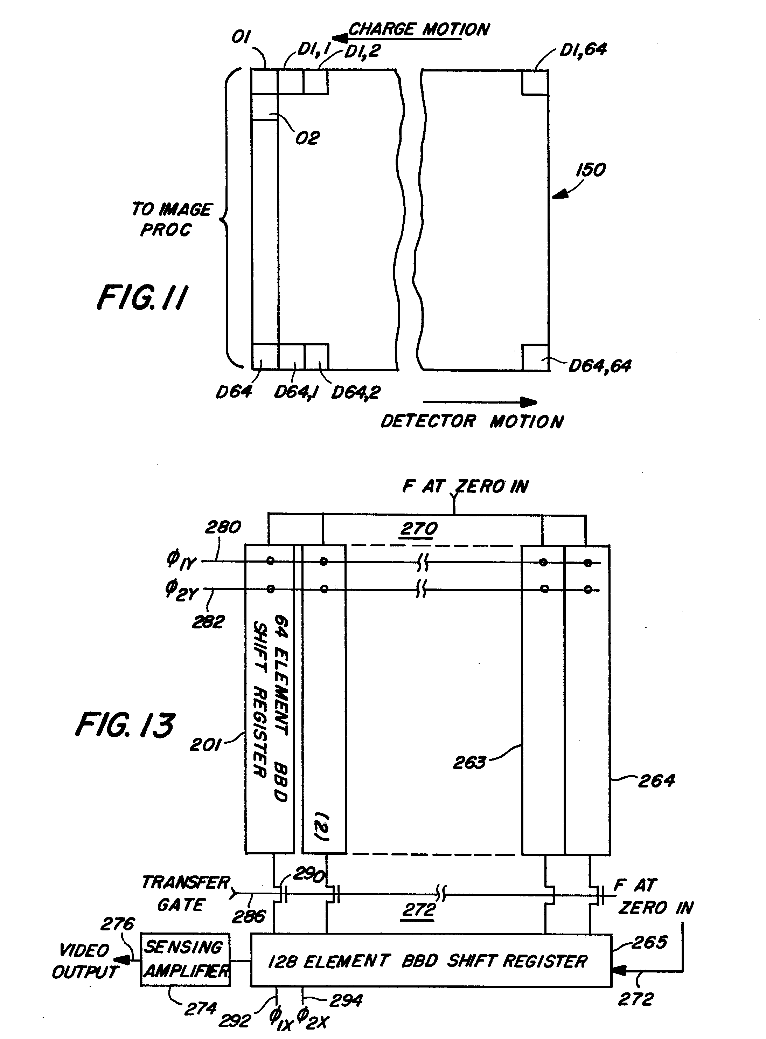

Figure 11 shows in more detail an embodiment of one of the imaging chips, indicated at reference character 150. In the Figure 11 embodiment, the image chip comprises a 64 x 64 rectilinear array of individual detector elements, each comprising a photodiode. The detector elements are arranged in 64 rows and columns.

For example, one such row comprises individual detector elements D1,1-D1,64. Sixty-four rows of detector elements are present on the imaging chip, extending downwardly to the row comprising individual detector elements D64,1-D64,64.

A shift register including cells O1-O64 is coupled in parallel to the column of detector elements D1,1-D64,1.

In use, the image chip, and the detector as a whole, move to the right as indicated by the arrow in Figure 11. During scanning motion, the shifting and integration circuitry cause accumulating charge motion in a direction to the left as shown in Figure 11, synchronously with the detector motion in the opposite direction. When the accumulated charge packets, each representing projections of the same area of the patient's body, have been so integrated and transferred and have reached the respective individual detector elements of column D1,1-D64,1, the charge packets are transferred in parallel to the shift register, i.e., into each of the cells O1-O64. The data thus accumulated can be clocked out, digitized, and processed to yield image representations in a manner known in the art. Systems for producing imaging data from sequentially occurring line-representing sets of data derived from a photodiode array are described in U.S. Patent No. 4,203,037, issued May 13, 1980 to Gur et al, particularly with respect to columns 6 and 7 of that patent, and the patents and publications incorporated therein. Patent No. 4,203,037, and all other patents and publications incorporated therein are specifically incorporated by reference here.

The 64 x 64 array of individual detector elements of the image chip 150 of Figure 11 collectively describe a square of 8 millimeters length on a side. As noted above, the detector 14 comprises a vertical column of such imaging chips, each being 8 millimeters wide, and the spread beam incident on the detector is vertically oriented and 8 millimeters in thickness, just sufficient to illuminate the entirety of the vertical column of imaging chips.

Figure 12 illustrates another embodiment of an image chip indicated by the reference character 170. The image chip 170 comprises a 64 x 256 array of rectangular individual detector elements. The array comprises 64 horizontal rows of 256 elements each. Each individual element has a horizontal width which is approximately 1/4 its vertical height. Together, the 64 x 256 detector array describes a square approximately 8 millimeters on a side.

As in the embodiment of Figure 11, the detector, during scanning, is moved to the right as shown in Figure 12, and the charge packets are shifted and integrated to the left, at a rate synchronous with that of detector motion.

The embodiment of Figure 12 is considered particularly suitable for the use of time delay and integrate circuitry consisting of CCD elements. The 64 x 64 array of Figure 11 is considered more suitable for the use of bucket brigade circuitry which can handle wider charge "bins", and hence has a greater capability than CCD for shifting charge packets efficiently. Efficiencies of at least about 0.99995 are desirable.

In operation, the charges are shifted to the left, as shown in Figure 11 in synchronism with detector scanning motion. Data respectively corresponding to projections of the same image line is integrated and transferred along each element until the charge reaches the extreme left-hand detector element in the leftmost column of detector elements, i.e. D1,1-D64,1. At that point, the charge packets are transferred and clocked successively through a series of parallel shift registers S1-S8. The clocking through the registers S1 through S8

is at the same rate as the clocking along the various detector elements.  No integration, however, takes place in the shift registers.

Preferably where, as in the case of the Figure 12 embodiment, the individual detector elements are only 1/4 as wide as they are tall, circuitry of known type is provided which, in conjunction with the shift registers S1-S8, periodically sums output data in the shift registers S1-S8.  After each succession of four shifts, the data in S1-S4, corresponding to each separate element row, is separately summed in appropriate output register circuitry, and then clocked out to digitization circuitry and image processing circuitry such as described in connection with Figure 11.

Figure 13 is a block diagram showing circuitry of the matrix array of an image chip in greater detail.

The image chip shown comprises a 64 x 64 element bucket brigade matrix array for detecting x-rays after they have been converted to light, similar to that of Figure 11.  The matrix array is operated in a time delay integration mode.  The center-to-center spacing of the individual detector elements in both the column and row directions is 5 mils.  The array is integrated using a double poly-silicon gate NMOS process.  The design details and dynamic range consideration of the image chip array are discussed in detail below.

The image chip includes 64 horizontal bucket brigade (BBD) shift registers 201-264.  Also included is a 128 element BBD shift register 265.

Each of the registers 201-265 is provided with a fat-zero input port (designated generally by reference characters 270, 272) for enhancing charge transfer efficiency in the registers.

Registers 201-264 perform the time delay and integrate (TDI) function while register 265 transfers the signal charge package to an output sensing amplifier 274. The output sensing amplifier converts the charge to a signal voltage appearing at a lead 276 which is a function of the charge input to the amplifier. The signal at the lead 276 provides a video output.

The center-to-center spacing of the registers 201-264 is 5 mils. Each of the registers 201-264 comprises 64 BBD elements (32 stages) and is driven by a two-phase complementary clock having inputs designated by the reference characters 280, 282.

Each BBD element is constructed with a diffused photodiode and a poly-silicon-to-poly-silicon storage capacitor. The sensing area of each photodiode is approximately 50% of the total area of the individual detector element receiving surface. The capacitance of each individual detector element is about 1.2 picofarads (pF).

When light falls upon each photodiode, photoelectrons are generated. This photocharge is stored on the storage capacitor. The two-phase clocks at the leads 280, 282 shift the charge packages along each of the shift registers 201-264 toward the register 265. In the TDI operation, the image velocity across the image chip is synchronized to the shift velocity of the charge package (integration site). The clocking rates of the signals at the leads 280, 282 is approximately 0.5 kilohertz (kHz). The total integration time of each pixel is approximately 64 milliseconds (msec).

This relatively slow integration time allows a tolerance for x-ray tube rotor and voltage ripple, and substantially reduces their artifacts.

When the charge packages reach the ends of the registers 201, 264 adjacent the register 265, a transfer pulse is applied to a transfer gate 295. This activates the transfer switches, shown for example at reference character 290.  The signal charges are thus transferred in parallel to the register 265 from each of the registers 201-264.

The register 265 is driven by a two-phase clock having inputs 292, 294.  This clock signal runs at a frequency 64 times faster than that of the clock signals at the leads 280, 282.

The output sensing amplifier of the register 265 is a gated charge integrator which converts the signal charges into output voltages.

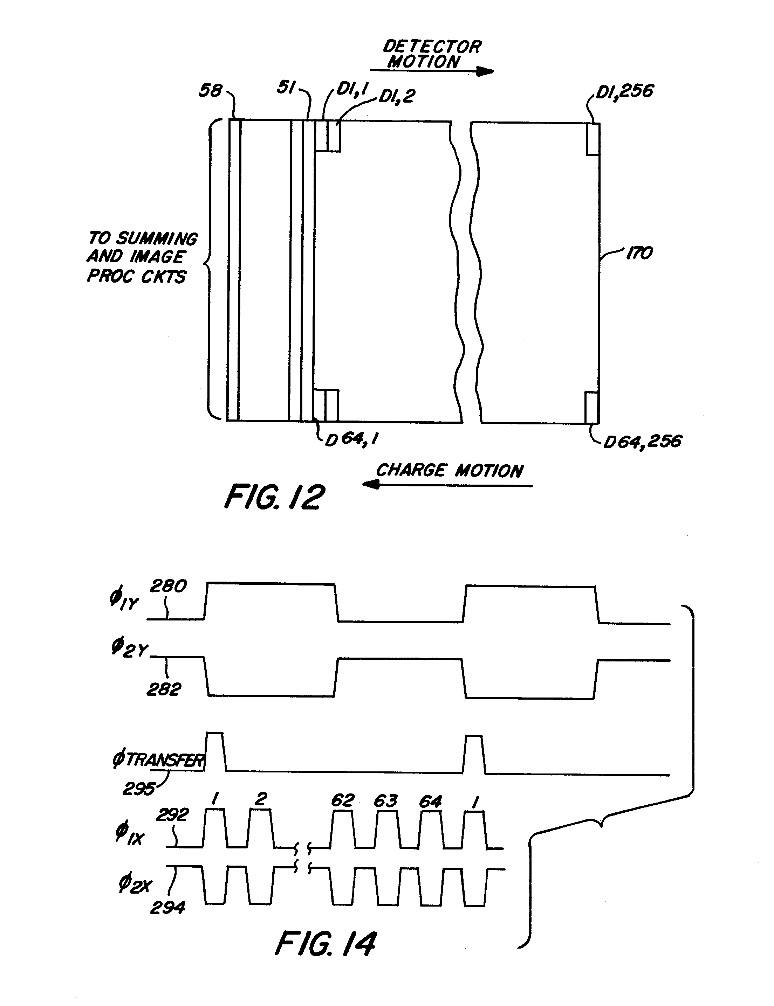

The timing diagram of the clocks is illustrated in Figure 14.

The clock waveforms 280, 282 and 292, 294 are complementary clocks, cross over at 50%.  The frequency $f_x$ = 64 X $f_y$ = 32 kHz.  The rising edge of waveform 292 leads that of waveform 295 by about 30 nanoseconds (ns.) and waveform 295 leads waveform 280 by about 20 ns. The falling edge of waveform 295 leads that of waveform 292 by at least 30 ns.

The charge handling capacity of the BBD elements depends upon the size of the storage capacitor and of the clock voltage.  Assuming a maximum voltage swing of 5 volts at each BBD storage node, the saturation charge of each BBD element will be about 6 picocoulombs (pC), i.e., 1.2 pF x 5 v.  This saturation charge is equivalent to a signal charge of 3.75 x $10^7$ electrons.

There are two noise components in the signal output from the sensing amplifier.  One is the fixed-pattern noise caused by nonuniformity of the input fat-zero among the registers 201-264.  This noise component can be eliminated by external signal processing.  Therefore it is not the limiting factor in the performance of the device.

The other noise component is the thermodynamic noise.  This noise consists of input kT noise, BBD transfer noise, dark current shot noise, photo signal shot noise and output amplifier noise. However, the dominant thermodynamic noise is BBD transfer noise.

At room temperature, the noise electron charge, due to this component, is approximately 6000 electrons. This noise figure results in a dynamic range of about 6250 ($3.75 \times 10^7/10^3$) for the device.

The calculated objective specifications of the matrix array is listed in Table I.

## TABLE I

Calculated Objective Specification of the Matrix Array.

| | |
|---|---|
| No. of Elements | 64 by 64 |
| Center-to-Center Spacing | |
| X Direction | 5 mils |
| Y Direction | 5 mils |
| Element Storage Capacitance | 1.2pF |
| Element Sensing Area | 50% of total element area |
| Saturation charge | 6 pC |
| Thermodynamic Noise | |
| Electron | $6 \times 10^3$ electrons |
| Dynamic Range due to Thermodynamic noise | 6000 |
| Saturation Output | Approx. 4V |

An example of time delay and integrate circuitry for use in imaging is described in the following article, which is expressly incorporated by reference: Milton, A. F. "Charge Transfer Devices for Infrared Imaging", pp. 197-228, which is a portion of the book "Optical and Infrared Detectors" edited by Keyes, R.J. (1977).

It is to be understood that the description of this invention set forth above is intended to be illustrative, rather than exhaustive, of the invention. It is to be recognized that persons of ordinary skill in

36

the relevant technical field will be able to make certain additions, deletions and modifications to the embodiments described herein without departing from the spirit or the scope of the invention, as defined in the appended claims.

CLAIMS

1. A radiographic system comprising: a source of penetrative radiation (10); a detector (14) having a plurality of energy sensitive detector elements for producing electrical signals in response to said radiation; and means for actuating the source to direct radiation through a subject (P) to emerge therefrom in a pattern a portion of which is incident on the detector (14); characterised in that it includes means (30) for moving the detector (14) relative to the subject (P) while simultaneously exposing a plurality of said detector elements to said radiation emergent from the subject (P); time delay and integrate circuitry associated with the detector elements for operating upon the electrical signals for enhancement of their signal to noise ratio, and imaging circuitry (44) responsive to said enhanced signals for producing a representation of internal subject structure corresponding to said pattern of emergent radiation.

2. A system according to Claim 1 wherein said means for moving the detector comprises means (30) for pivoting the radiation source (10) and for moving the detector (14) in synchrony with said pivoting motion for simultaneously exposing a plurality of said detector elements to said radiation emergent from the subject (P).

3. A system according to either of the preceding claims wherein said detector has a plurality of light sensitive detector elements (86) for producing electrical signals in response to incident light energy, said detector further comprising: means (96) for converting the penetrative radiation to light energy, and means (90) for optically coupling said converting means to said detector elements (86); and said means for moving the detector (14) relative to the subject (P) simultaneously exposes a plurality of the detector elements to said converted light energy corresponding to the pattern of penetrative radiation emergent from the subject (P).

4.    A system according to Claim 3 in which said light sensitive detector elements (86) do not face said source of penetrative radiation (10).

5.    A system according to Claim 3 or Claim 4 in which said means for optically coupling comprises a fibre optic light pipe (90).

6.    A system according to any one of the preceding claims in which said means for actuating the source (10) to direct penetrative radiation through the subject (P) to emerge therefrom in a pattern causes a portion of such emergent pattern to be directly incident on the detector (14) without intermediate processing or conversion.

7.    A system according to Claim 1 in which said source is an x-ray source (10) and said detector is an x-ray detector (14) including a first elongated array of detector elements each element responsive to occurrence of x-ray energy incident on a different detector region to produce and maintain an electrical signal representing the incident x-ray energy; and said means for moving the detector comprises a scanner apparatus (24) comprising: apparatus for effecting longitudinal motion of the detector element array at a predetermined velocity relative to the subject (P) to be examined; and apparatus (30) associated with the x-ray source (10) for directing x-ray energy through the subject (P) to emerge for simultaneously exposing to x-ray a plurality of said detector elements during the course of said longitudinal relative motion; and said time delay and integrate circuitry comprises circuitry (56) coupled to the detector elements for shifting and integrating said electrical signals iteratively in a longitudinal direction along the detector elements at velocity equal and opposite to said predetermined velocity of longitudinal detector motion and output circuitry (44) for transmitting said shifted and integrated electrical signals from a circuitry location associated with one of said detector elements synchronous with each arrival of a shifted and integrated signal at said circuitry location.

8. A system according to Claim 7, further comprising: structure (16) defining a fore slit (18) interposed between the x-ray tube (10) and the subject (P) for collimating x-ray energy emanating from the tube; structure (20) defining an aft slit (22) positioned between the subject (P) and said detector array for further collimating said x-ray energy, and said scanner apparatus (24) further comprising: apparatus for moving said structures defining said fore (18) and aft (22) slits synchronously with said pivoting motion of said x-ray tube (10) to maintain said slits continuously in a beam of x-ray energy emanating from the tube and for maintaining mutual alignment of the slits with the detector (14).

9. A system according to Claim 7 or Claim 8 further comprising: an encoder (36) for sensing location of the movable detector array and coupled to synchronize said x-ray tube pivoting motion with motion of the detector array.

10. A system according to anyone of Claims 7 to 9 wherein said detector array comprises: a plurality of imaging chips ( 70, 72, 74) each imaging chip including a rectangular matrix of individual detector elements, said matrix comprising additional rows of detector elements parallel to said first detector element array, said imaging chips being arranged in a column whose longitudinal dimension is substantially perpendicular to the longitudinal dimension of said first detector element array, and additional shifting and integrating circuitry associated with each additional row of detector elements extending parallel to said longitudinal dimension of said first array of detector elements.

11. A system according to any one of Claims 7 to 10 wherein said shifting and integrating circuitry comprises: a different storage capacitor ($C_3$, $C_2$, $C_1$) associated with each of said detector elements ($X_3$, $X_2$, $X_1$); circuitry (SR) defining a shift register connecting adjacent storage capacitors along the longitudinal dimension of said first array, and clocking circuitry (56) for iteratively step shifting and integrating electric charge packets stored in said storage capacitors.

12. A system according to any one of Claims 7 to 11 wherein said shifting and integrating circuitry comprises bucket brigade circuitry.

13. A radiographic system according to any one of the preceding claims in which said time delay and integrate circuitry comprises shift register (201-265) and clocking circuitry (46) associated with the detector elements for operating upon the electrical signals for enhancement of their signal to noise ratio, said time delay and integrate circuitry comprising: means (295) associated with the clocking circuitry for periodically reversing the direction of clocking of charge signals through the shift register circuitry; means (290) for reversing the direction of said detector movement in synchrony with said reversed clocking, and circuitry coupled for reading data out of either end of said shift register dependent on the direction of said clocking and said detector movement.

14. A radiographic system comprising: a source for directing penetrative energy through a subject; a detector including an array of individual detector elements, each detector element having a rectangular shaped receiving face facing toward said source, a width dimension of each said detector element face being substantially less than an orthongonal length dimension of said detector element, each said detector element responsive to the occurrence of incident emanation to produce electrical signals representing said emanations; and imaging circuitry coupled to said detector elements for processing said electrical signals and producing from said processed signals a tangible representation of internal subject structure.

15. An x-ray detector assembly comprising: a portion of material (96) responsive to x-ray energy incident thereon to produce visible light; an array of individual detector elements (98); each element having a receiving face optically coupled to said material and each element being responsive to visible light incident on its receiving surface to produce an electrical signal representing such light, characterised in that each element is disposed at a location such that its receiving face does not directly face the x-ray energy.

16. An assembly according to Claim 15 in which each element is optically coupled to said material by an optical fibre (90).

17. A method of using a radiographic system having an x-ray source (10) and an x-ray detector (14) including a linear array of individual detector elements each element responsive to incidence of x-rays in a different region to produce and maintain an electrical signal representing the x-ray, said method being characterised in that it includes the steps of: effecting longitudinal detector motion of a predetermined velocity relative to a subject to be examined; by pivoting the x-ray source (10) about an x-ray focal spot; and moving said detector array (14) synchronously with said pivoting of said source to maintain at least a portion of the detector array continuously in a beam of x-rays emitted by said source; directing x-rays simultaneously to regions of said detector corresponding to a plurality of detector elements during said motion to cause the generation of said electrical signals; shifting and integrating said electrical signals along said detector at velocity equal and opposite to said predetermined velocity of detector motion relative to the subject; transmitting said shifted and integrated signals from said detector in response to the arrival of each shifted and integrated signal at a predetermined location, and processing said transmitted signals to produce a representation of internal subject condition.

18. The method of Claim 17, utilizing collimating structure (16) defining a fore slit (18) interposed between the source (10) and the subject (P) and structure (20) defining an aft slit (22) positioned between the subject (P) and the detector (14), said method comprising the additional step of: moving said slit defining structures synchronously with said pivoting of said x-ray source to maintain said slits continuously in a beam of x-ray energy from the source and for maintaining mutual alignment of the slits.

FIG. 1

FIG. 2

0138625

2 / 6

FIG. 3

$t=T_1$

CHARGE SHIFT

FIG. 4A

$t=T_2$

FIG. 4B

$t=T_3$

FIG. 4C

$t=T_4$

FIG. 4D

0138625

3 / 6

FIG. 5

FIG. 6

FIG. 7

RADIATION IN

RADIATION SENSITIVE
PHOSPHOR OR
SCINTILLATO

CHARGE COUPLED
IMAGE CHIPS ARE
MOUNTED ON FACES
OF FIBER

FIG.8

FIG. 9

FIG. 10

FIG. 11

**CHARGE MOTION**

O1, D1,1, D1,2, D1,64, O2, 150

TO IMAGE PROC

D64, D64,1, D64,2, D64,64

**DETECTOR MOTION**

FIG. 13

F AT ZERO IN

270, 280, $\phi_{1Y}$, $\phi_{2Y}$, 282, 201

64 ELEMENT BBD SHIFT REGISTER

263, 264, (2)

TRANSFER GATE, 290, 286, 272, F AT ZERO IN, 265

276, VIDEO OUTPUT, SENSING AMPLIFIER, 128 ELEMENT BBD SHIFT REGISTER

274, 292, $\phi_{1X}$, $\phi_{2X}$, 294, 272

DETECTOR
MOTION →

58   51   DI,1  DI,2                          DI,256

TO SUMMING
AND IMAGE
PROC CKTS

170

D 64,1                                        D 64,256

← CHARGE MOTION

## FIG. 12

$\phi_{1Y}$ 280

$\phi_{2Y}$
282

$\phi_{TRANSFER}$
295

1  2      62  63  64  1
$\phi_{1X}$ 292

$\phi_{2X}$ 294

## FIG. 14